# EUROPEAN PATENT APPLICATION

(11) **EP 4 067 494 A1**
(43) Date of publication of application: **05.10.2022**
(21) Application number: 20892590.9
(22) Date of filing: 23.11.2020
(51) Int. Cl.: C12N 15/85, C12N 5/0793, C07K 14/47, C12Q 1/02, G01N 33/50, A61K 48/00, A61P 25/00

(54) **IMPROVED IN VIVO REPROGRAMMING SYSTEM AND CELL CONVERSION METHOD USING SAME**

(30) Priority: 25.11.2019 KR 20190152040
(71) Applicant: Industry-Academic Cooperation Foundation, Yonsei University, Seoul 03722 (KR)
(72) Inventor: HA, Yoon, Seoul 03722 (KR); LEE, Hye Lan, Seoul 03786 (KR); LEE, Hye Yeong, Seoul 03786 (KR)
(74) Representative: Schnappauf, Georg
(86) International application number: PCT/KR2020/016578
(87) International publication number: WO 2021/107530

(57) **Abstract**

The present disclosure relates to an advanced *in vivo* reprogramming system and a cell conversion method using same. The reprogramming system of the present disclosure comprises a start cell marker promoter, a pluripotency-maintaining gene protein, an amino acid isolation peptide, Cre recombinase, a target cell marker promoter, LoxP, and a gene encoding a fluorescent protein, does not require cell fixation in order to confirm cell conversion, enables real-time monitoring in a living cell state, and may be used both *in vitro* and *in vivo.* Therefore, the present disclosure is expected to be widely used in the biological and medical fields.

## Description

### Technical Field

The present disclosure relates to an advanced *in vivo* reprogramming system and a cell conversion method using the same.

### Background Art

Recently, in the field of stem cell research, as pluripotency maintaining gene proteins such as SOX2 (Sex determining region Y-box 2) have been identified, efforts have been made to convert completely differentiated cells into other types of cells by overexpressing the pluripotency maintaining gene protein in the completely differentiated cells. For example, Huang et al. conducted a study on converting normal somatic cells into hepatocytes using Foxa3, Hnfla and Gata4 genes (Huang et al., Nature, vol.475, pp.386-389, 2011), and Korean Patent No. 10-1702629 discloses a method of converting normal somatic cells into vascular progenitor cells using FLI1 or ETV2 gene. Unlike the existing process of reprogramming completely differentiated cells into induced pluripotent stem cells (iPSCs) and re-differentiating the reprogrammed cells into desired cells, this method of converting completely differentiated cells directly into other types of cells induces the conversion of the completely differentiated cells directly into desired cells without the induced pluripotent stem cell stage, and thus is receiving attention for its potential to be used for disease modeling and discovery of new drugs, and is also expected to be highly useful in the field of regenerative medicine.

However, conventional known cell conversion methods have problems in that the efficiency of converted cells is quite low and there is no technology capable of monitoring cell conversion in real time. Since it is impossible to measure the state of cells in real time, it is not possible to determine the exact time until cell conversion is completed, and in order to confirm cell conversion, it is necessary to fix the cells and then investigate the cells by an immunological method. Thus, problems arise in that it is impossible to use the converted cells secondarily and it is possible to perform cell conversion only *in vitro.*

Accordingly, the present disclosure relates to an advanced *in vivo* reprogramming system and a cell conversion method using the same. The reprogramming system of the present disclosure does not require cell fixation to confirm cell conversion, enables real-time monitoring in a living cell state, and may be used both *in vitro* and *in vivo.* Thus, the reprogramming system of the present disclosure is expected to be widely used in the biological and medical fields.

### DISCLOSURE

### Technical Problem

The present disclosure has been made in order to solve the above-described problems occurring in the prior art, and relates to an advanced *in vivo* reprogramming system and a cell conversion method using the same.

However, the technical problem to be achieved by the present disclosure is not limited to the technical problem mentioned above, and other technical problems not mentioned herein will be clearly understood by those of ordinary skill in the art from the following description.

### Technical Solution

Hereinafter, various embodiments described herein will be described with reference to the accompanying drawings. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, known processes and preparation techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise stated in the present specification, all the scientific and technical terms used in the specification have the same meanings as commonly understood by those skilled in the technical field to which the present invention pertains.

The present disclosure provides an advanced *in vivo* reprogramming system. The *in vivo* reprogramming system of the present disclosure is produced by introducing VSV-G (fusiogenic envelope G glycoprotein of the vesicular stomatitis virus), GAG/Pol gene, and an expression vector into human embryonic kidney 293FT (HEK293FT) cells using a Viafect transfection reagent (E4981, Promega, USA). HEK293FT cells are cells derived from human fetal kidney cells, and are actively used in the field of gene research because of their high growth rate and high transfection efficiency.

The expression vector was designed to sequentially transcribe a starting cell marker promoter, a pluripotency maintaining gene protein, an amino acid isolation peptide, Cre recombinase, a target cell marker promoter, LoxP, and a gene encoding a fluorescent protein. The fluorescent protein was designed in the direction of reverse transcription so as to recognize LoxP by the expression of Cre recombinase and to restore in the direction of transcription.

In the present specification, the starting cell marker promoter is, in a strict sense, a promoter of a gene encoding a marker protein that is expressed specifically in a starting cell, and the target cell marker promoter is in a strict sense, a promoter of a gene encoding a marker protein that is expressed specifically in a target cell.

The starting cell is a completely differentiated cell into which the expression vector is introduced, and is a cell expressing a marker promoter located first in the transcriptional direction of the expression vector. The starting cell may be selected from any type of cells, and any type of completely differentiated somatic cell or germ cell may be selected as the starting cell.

The target cell is a cell to be converted from the starting cell, and is a cell expressing a marker promoter located second in the transcriptional direction of the expression vector. The target cell may also be selected from any type of cells, and any completely or incompletely differentiated somatic cell or germ cell may be selected as the target cell.

Specifically, in one embodiment, the starting cell and the target cell may be selected from subtypes of epithelial cells, muscle cells, neurons, stromal cells, and bone cells.

The epithelial cells are cells constituting a membrane tissue that forms a layer of cells and covers the inside and outside of a tissue or gland. The epithelial cells are meant to encompass simple squamous epithelium, simple cuboidal epithelium, simple columnar epithelium, stratified squamous epithelium, stratified cuboidal epithelium, stratified columnar epithelium, pseudostratified epithelium, transitional epithelium, and glandular epithelium. The muscle cells are cells constituting the muscle, and are meant to encompass smooth muscle cells, striped muscle cells, and myocardial cells.

The neurons are cells capable of signaling in an electrical way by expressing ion channels such as a sodium channel and a potassium channel, unlike other cells, and are meant to encompass sensory neurons, interneurons, motor neurons, unipolar neurons, pseudo-unipolar neurons, bipolar neurons, multipolar neurons, spiny neurons, aspinous neurons, non-spiny neurons, cholinergic neurons, adrenergic neurons, dopaminergic neurons, and serotoninergic neurons.

The stromal cells are cells constituting the fibrous connective tissue, and are meant to encompass fibroblasts, chondroblasts, osteoblasts, neuroglial cells, adipocytes, macrophages, and plasma cells.

The bone cells are meant to encompass osteoblasts, osteocytes, and osteoclasts.

In another embodiment, the starting cell and the target cell may be selected from cells constituting the skeletal system, the muscular system, the endocrine system, the circulatory system, the urinary system, the reproductive system, the digestive system, the nervous system, the skin system, the respiratory system, and the exocrine system.

The skeletal system is meant to encompass bones including a body skeleton (skull, spine, and chest cage), a limb skeleton (arm skeleton, and leg skeleton), bone marrow, etc., cartilages including hyaline cartilage, fibrocartilage, elastic cartilage, etc., and joints including fibrous joints, cartilaginous joints, synovial joints, etc.

The muscular system is meant to encompass skeletal muscle, smooth muscle, and cardiac muscle.

The endocrine system is meant to encompass the pituitary gland, hypothalamus, pineal gland, thyroid gland, parathyroid gland, thymus, adrenal gland, pancreas, testis, and ovary.

The circulatory system is meant to encompass the cardiovascular system including the heart, blood vessels (arteries, veins, capillaries, aorta, vena cava, pulmonary artery, and pulmonary vein), blood (plasma, red blood cells, white blood cells, and platelets), etc., and the lymphatic system including lymphatic vessels, lymph nodes, spleen, thymus, tonsils, Peyer's plate, mucosa-associated lymphoid tissues.

The urinary system is meant to encompass the kidneys, ureters, bladder, and urethra.

The reproductive system is meant to encompass the male reproductive system including testes, epididymis, vas deferens, spermatozoa, urethra, seminal vesicle, prostate gland, bulbous urethral gland, scrotum, penis, glans, foreskin, etc., and the female reproductive system including ovaries, uterine tubes, uterus, vagina, hymen, vulva, labia majora, labia minora, and mammary glands.

The digestive system is meant to encompass the digestive tract including the mouth, pharynx, esophagus, stomach, small intestine (duodenum, jejunum, and ileum), large intestine (caecum, colon, and rectum), anus, etc., and the digestive glands including salivary glands, pancreas, gallbladder, liver, etc.

The nervous system is meant to encompass the central nervous system (brain, and spinal cord), peripheral nervous system (body nervous system), autonomic nervous system (sympathetic nervous system, and parasympathetic nervous system)), eyes, ears, nose, and tongue.

The skin system is meant to encompass skin, hair follicles, sweat glands, sebaceous glands, nails, and breasts.

The respiratory system is meant to encompass the nasal cavity, pharynx, larynx, trachea, bronchi, and lungs.

The exocrine system is meant to encompass sweat glands, mandibular urethral glands, skin glands, mammary glands, prostate glands, anterior osseous glands, mucus, seminal vesicles, salivary glands, and estuary glands.

In addition, as the starting cell marker promoter and the target cell marker promoter in the *in vivo* reprogramming system of the present disclosure, any promoters may be used without limitation as long as they are the promoters of the genes encoding proteins known as selected cell markers to those skilled in the art.

Hereinafter, the starting cell will be referred to as cell A, and the target cell will be referred to as cell B.

In an embodiment of the present disclosure, a stromal cell was selected as cell A, and a neuron was selected as cell B.

More specifically, the stromal cells are cells constituting the fibrous connective tissue, and are meant to encompass fibroblasts, chondroblasts, osteoblasts, neuroglial cells, adipocytes, macrophages, and plasma cells, and the neuroglial cells are meant to encompass astrocytes, oligodendrocytes, microglia, ependymal cells, Schwann cells, satellite cells, and capsular cells.

When cell A is a stromal cell, the cell A marker selected may be Col1A2 (Collagen Type I Alpha 2 Chain), FAP (fibroblast-activation protein), FSP1 (fibroblast-specific protein 1), Vimentin, ACTA (alpha smooth muscle actin), Hsp47 (heat shock protein 47), aggrecan, CD44, CD45, CD73, calcitonin, OSCAR (osteoclast-associated receptor), RANK (receptor activator of nuclear factor κ B), GFAP (glial fibrillary acidic protein), TREM2 (triggering receptor expressed on myeloid cells 2), HexB (beta-hexosaminidase subunit beta), S100 (calcium-binding protein), CD69, and/or Gpr34 (probable G-protein coupled receptor 34), but is not limited thereto.

Cell B is a neuron, the cell B marker selected may be SYN (synapsin), Tuj 1 (neuron-specific class III beta-tubulin), MAP2 (microtubule-associated protein 2), and/or neurofilament, but is not limited thereto.

In one embodiment of the present disclosure, the cell A marker promoter may be a GFAP (glial fibrillary acidic protein) promotor, the pluripotency maintaining gene protein may be SOX2 (sex determining region Y-box 2), the amino acid isolation peptide may be T2A (2A peptide), the cell B marker promoter may be SYN (synapsin), and the fluorescent protein may be eGFP (enhanced green fluorescent protein). In this case, cell A is an astrocyte, and cell B converted from the astrocyte by the expression vector of the present disclosure is a neuron. When the above exemplary vector is introduced into astrocytes, SOX2 and Cre recombinase are expressed by the GFAP promoter, which is an astrocyte marker, and the expression of SOX2, which is a pluripotency maintaining gene protein, may convert the astrocytes into neurons. In addition, the LoxP sites are recognized by Cre recombinase, and the fluorescent protein in the direction of reverse transcription between the Loxp sites is restored in the direction of transcription. When the astrocytes are converted into neurons, the SYN promoter, which is a neural marker, works, and eGFP restored in the direction of transcription is expressed by the SYN promoter, and the cells display green fluorescence. As a result, cell B (neurons) converted from cell A (astroglia) may be identified as living cells *in vivo.*

If the expression vector is introduced into cells other than cell A (astrocytes), the cell A marker promoter (GFAP promoter) does not work, and thus no fluorescence is displayed. Even if the expression vector is introduced into cell A (astrocytes), if cell A is converted into cells other than cell B (neurons) by the pluripotency maintaining protein (SOX2) gene, the cell B marker promoter (SYN promoter) does not work, and thus no fluorescence is display. If the expression vector is introduced into cell B (neurons) from the beginning, Cre recombinase is not expressed, and thus fluorescence is not displayed due to the failure of Cre-loxP working. Only when cell A (astroglia) is converted to cell B (neurons), fluorescence is displayed.

The T2A peptide is a self-processing viral peptide having a relatively short amino acid sequence consisting of about 18 amino acids. When the 2A sequence is placed between two different proteins and expressed in cells after fusion, the amino acid bond is broken in front of the last amino acid Pro of the 2A sequence, and thus the same effect as if the two proteins were expressed separately may be obtained. As a kind of 2A, F2A or E2A may also be used as a substitute.

The Cre-loxP system is a site-specific recombination system that may selectively delete, invert or translocate a specific gene. Unlike other vectors in which a gene is expressed immediately after transformation, the Cre-loxP system may induce expression of the inserted gene only in a necessary tissue at a necessary time. When loxP is positioned before and after the gene to be manipulated and Cre recombinase is expressed, the target gene positioned between the loxPs is manipulated by Cre-loxP interaction (https://www.addgene.org/crelox/).

The advanced *in vivo* reprogramming system of the present disclosure is receiving attention for its potential to be used for disease modeling and discovery of new drugs, and is also expected to be highly useful in the field of regenerative medicine.

For example, when a specific type of cell is set as cell A and cell A is removed by converting it to cell B, it is possible to model what kind of disease occurs due to the absence of cell A. For example, when a specific disease is treated by converting cell A into cell B to increase the number of cells B in a subject with the disease, cell B may be used for pharmaceutical purposes for the disease.

In addition, for example, in the case of completely differentiated cells that have hardly regenerated, the regeneration of function may be induced by converting other cells (cell A) into target cells (cell B). That is, the loss of neural function after damage may be restored. More specifically, since neurons hardly regenerate after damage, sensory and motor impairments may occur upon nerve injury. However, it is possible to restore lost neural function by converting other cells (cell A) into neurons (cell B).

The advanced *in vivo* reprogramming system of the present disclosure enables live cell imaging of the process of converting specific cells, set as cell A, into cell B in a living cell state. Since it does not require cell fixation, it is possible to use the converted cells secondarily, and it is possible to check the process of cell conversion from cell A into cell B in real time without a time lapse. Preferably, the live cell imaging is performed using a fluorescence microscope.

In one embodiment of the present disclosure, the term "diagnosis" refers to identifying the presence or characteristics of a pathological condition, identifying the occurrence of a disease, and determining the severity of the disease. To this end, a suspected disease may be diagnosed by visual or cytological examination of a tissue from a subject suspected of having the disease. Specifically, the disease may be diagnosed by a method of measuring the concentration of lipids contained in a sample (clinically, cells, blood, fluid, pleural fluid, ascites, joint fluid, pus, secretion, sputum, pharyngeal mucus, urine, bile, feces, etc.) from a subject suspected of having the disease, a method of directly detecting a disease-related protein in the sample, a method of measuring the concentration of an antibody specific for the protein, or a method of directly detecting a nucleic acid encoding the protein and/or the antibody. Examples of the method for measuring lipids include, but are not limited to, staining with a dye that reacts specifically with lipids. Examples of diagnostic means using the method of directly detecting antigen-antibody binding or disease-related protein include, but are not limited to, Western blot analysis, ELISA (enzyme linked immunosorbent assay), radioimmunoassay (RIA), radioimmunodiffusion, Ouchterlony immunodiffusion, rocket immunoelectrophoresis, tissue immunostaining, immunoprecipitation assay, complement fixation assay, fluorescence activated cell sorter (FACS), and protein chip assay. Examples of the method of directly detecting a nucleic acid encoding a disease-related protein include, but are not limited to, reverse transcription polymerase reaction (RT-PCR), competitive reverse transcription polymerase reaction (competitive RT-PCR), real-time reverse transcription polymerase reaction (real-time RT-PCR), RNase protection assay (RPA), Northern blotting, or DNA chip assay.

In one embodiment of the present disclosure, "screening" refers to selecting a target substance having any specific property from a candidate group consisting of several substances by a specific manipulation or evaluation method. For the purposes of the present disclosure, screening in the present disclosure is a cell B tracking process in which disease treatment occurs by converting cell A into cell B to increase the number of cell B in a subject with a specific disease, or screening is a process of tracking cell A which is most effectively converted into cell B, but screening is not limited thereto.

In one embodiment of the present disclosure, "administration" means introducing a specific composition to a subject by any suitable method, and the composition may be administered through any general route as long as it may reach the target tissue. Examples of the administration route include, but are not limited to, oral administration, intraperitoneal administration, intravascular administration, intramuscular administration, subcutaneous administration, intradermal administration, intranasal administration, intrapulmonary administration, intrarectal administration, intrathecal administration, intraperitoneal administration, intrathecal administration. In the present disclosure, the effective amount may be adjusted depending on various factors, including the type of disease, the severity of the disease, the types and contents of the active ingredient and other ingredients contained in the composition, the type of formulation, the subject's age, weight, general health status, sex and diet, the time of administration, the route of administration, the secretion rate of the composition, the duration of treatment, and concurrently used drugs. For adults, the composition may be administered into the body in an amount of 50 ml to 500 ml at a time, the compound may be administered at a dose of 0.1 ng/kg to 10 mg/kg, and a monoclonal antibody against a protein may be administered at a dose of 0.1 ng/kg to 10 mg/kg. Administration may be performed once to 12 times a day, and when administration is performed 12 times a day, administration may be performed once every 2 hours. Peptides and nucleic acids may also be administered in combination with other treatments designed to enhance immune responses, e.g., by co-administration with adjuvants or cytokines (or nucleic acids encoding cytokines), as is well known in the art. Other standard delivery methods, e.g., biolistic transfer or ex vivo treatment, may also be used. In *ex vivo* treatment, antigen presenting cells (APCs) such as dendritic cells, peripheral blood mononuclear cells, or bone marrow cells may be obtained from a patient or an appropriate donor and activated *ex vivo* with the immunogenic compositions, and then administered into the patient.

One embodiment of the present disclosure provides an expression vector for converting cell A into cell B, the expression vector sequentially containing a cell A marker promoter, a pluripotency maintaining gene protein, Cre recombinase, a cell B marker promoter, LoxP, and a gene encoding a fluorescent protein. The expression vector is capable of confirming that cell A is converted to cell B in a living cell state. In the expression vector, the gene encoding the fluorescent protein is contained in the direction of reverse transcription. The expression vector further contains an amino acid isolation peptide. In the expression vector, the amino acid isolation peptide is any one or more selected from the group consisting of T2A, F2A, and E2A. In the expression vector, the pluripotency maintaining gene protein is any one or more selected from the group consisting of a SOX gene family (sex determining region gene family), a Myc gene family (proto-oncogene gene family), a Klf gene family (Kruppel-like factors gene family), and an Oct gene family (octamer-binding transcription factor gene family).

In addition, in the expression vector, cell A is a stromal cell, and cell B is a neuron. In the expression vector, the stromal cell is any one selected from the group consisting of fibroblasts, chondroblasts, osteoblasts, neuroglial cells, adipocytes, macrophages, and plasma cells, and the cell A marker promoter is any one promoter selected from the group consisting of Col1A2 (Collagen Type I Alpha 2 Chain), FAP (fibroblast-activation protein), FSP1 (fibroblast-specific protein 1), vimentin, ACTA (alpha smooth muscle actin), Hsp47 (heat shock protein 47), aggrecan, CD44, CD45, CD73, calcitonin, OSCAR (osteoclast-associated receptor), RANK (receptor activator of nuclear factor κ B), GFAP (glial fibrillary acidic protein), TREM2 (triggering receptor expressed on myeloid cells 2), HexB (beta-hexosaminidase subunit beta), S100 (calcium-binding protein), CD69, and Gpr34 (probable G-protein coupled receptor 34). In the expression vector, the cell B marker promoter is any one promoter selected from the group consisting of SYN (synapsin), Tuj 1 (neuron-specific class III beta-tubulin), MAP2 (microtubule-associated protein 2), and Neurofilament.

Another embodiment of the present disclosure provides a pharmaceutical composition for treating nerve injury containing the expression vector as an active ingredient.

Still another embodiment of the present disclosure provides a lentiviral vector containing the expression vector, VSV-G (fusiogenic envelope G glycoprotein of the vesicular stomatitis virus) and a GAG/Pol gene.

Yet another embodiment of the present disclosure provides a non-human transformant containing the expression vector.

Still yet another embodiment of the present disclosure provides a method for converting cell A into cell B, the method including steps of: (a) producing the expression vector; and (b) introducing the expression vector into cell A. The method for converting cell A into cell B further includes a step of culturing the cell with a cell B culture medium, after step (b). In addition, the method further includes a step of confirming fluorescence expression in the cell, after step (b).

Another embodiment of the present disclosure provides a live cell imaging method for confirming the conversion of cell A into cell B in a living cell state, the method including steps of: (a) producing the expression vector; and (b) introducing the expression vector into cell A. In the live cell imaging method, the imaging is performed using a fluorescence microscope.

Another embodiment of the present disclosure provides a method for producing an animal model in which cell A has been converted into cell B, the method including step of: (a) producing the expression vector; and (b) introducing the expression vector into a non-human subj ect.

Another embodiment of the present disclosure provides a method for screening cell A for conversion into cell B, the method including steps of: (a) preparing a first subject and a second subject as a non-human disease animal model; (b) producing expression vector I by selecting cell (i) as cell A and cell (iii) as cell B; (c) producing expression vector II by selecting cell (ii) as cell A and cell (iii) as cell B; (d) introducing expression vector I into the first subject, and introducing expression vector II into the second subject; and (e) comparing a disease therapeutic effect between the first subject and the second subject, and selecting cell (i) as a cell for conversion into cell (iii) when the therapeutic effect on the first subject is better.

Another embodiment of the present disclosure provides a pharmaceutical composition for treating a disease caused by damage to cell B, the pharmaceutical composition containing the vector as an active ingredient, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.

Another embodiment of the present disclosure provides a method of preventing or treating a disease caused by damage to cell B by administering the vector, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.

Another embodiment of the present disclosure provides the use of the above-described vector for preventing or treating a disease caused by damage to cell B, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.

Hereinafter, each step of the present disclosure will be described in detail.

### Advantageous Effects

Conventional known cell conversion methods have problems in that, because there is no technology capable of monitoring cell conversion in real time, it is impossible to measure the state of a cell in real time, and it is possible to perform cell conversion only *in vitro.* Accordingly, the present disclosure relates to an advanced *in vivo* reprogramming system and a cell conversion method using the same. The reprogramming system of the present disclosure enables real-time monitoring in a living cell state, and may be used both *in vitro* and *in vivo.* Thus, the reprogramming system of the present disclosure is expected to be widely used in the biological and medical fields.

### Brief Description of Drawings

FIG. 1 is a view showing the structure of an advanced *in vivo* reprogramming system (A-IVR), which is an expression vector for direct reprogramming according to one embodiment of the present disclosure.
FIG. 2 is a view showing the results of immunostaining of neuroglial cells, more specifically astrocytes, converted into neurons by A-IVR *in vitro* according to one embodiment of the present disclosure.
FIG. 3 shows optical micrographs and the results of immunostaining of mouse embryonic fibroblasts, converted into neurons by A-IVR *in vitro* according to one embodiment of the present disclosure.
FIG. 4 shows optical micrographs and the results of immunostaining of human fibroblasts, converted into neurons by A-IVR *in vitro* according to one embodiment of the present disclosure.
FIG. 5 shows results indicating that the spinal cord injury animal model transplanted with A-IVR according to one embodiment of the present disclosure has recovered the motor function thereof.
FIG. 6 shows immunostaining results for the spinal cord tissue of the spinal cord injury animal model transplanted with A-IVR according to one embodiment of the present disclosure.

### Best Mode

In one embodiment of the present disclosure, neuroglial cells and neurons are selected as starting cells and target cells, respectively, and the neuroglial cells are converted into the neurons. In this case, a GFAP (glial fibrillary acidic protein) promoter is selected as a marker promoter for the starting cells, and a SYN (synapsin) promoter is selected as a marker promoter for the target cells. In another embodiment of the present disclosure, fibroblasts cells and neurons are selected as starting cells and target cells, respectively, and the fibroblasts are converted into the neurons. In this case, a Col1A2 (Collagen Type I Alpha 2 Chain) promoter is selected as a marker promoter for the starting cells.

### Mode for Invention

Hereinafter, the present disclosure will be described in more detail with reference to examples. These examples serve merely to explain the present disclosure in more detail, and it will be apparent to those of ordinary skill in the art that the scope of the present disclosure according to the subject matter of the present disclosure is not limited by these examples.

### Example 1. Construction of Lentiviral Vector for In Vivo Reprogramming

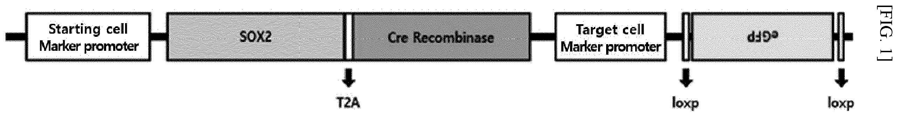

VSV-G (fusiogenic envelope G glycoprotein of the vesicular stomatitis virus), GAG/Pol gene and an expression vector were introduced into HEK293FT (human embryonic kidney 293FT) cells using a Viafect transfection reagent (E4981, Promega, USA). The expression vector was designed to sequentially transcribe a starting cell marker promoter, SOX2 (sex determining region Y-box 2), T2A (2A peptide), Cre recombinase, a target cell marker promoter, LoxP, and eGFP (enhanced green fluorescent protein), and the eGFP was designed in the direction of transcription so as to be reversed in the direction of transcription by LoxP expression. The gene structure of the expression vector is shown in FIG. 1.

For example, in one embodiment of the present disclosure, neuroglial cells and neurons are selected as starting cells and target cells, respectively, and the neuroglial cells are converted into the neurons. In this case, a GFAP (glial fibrillary acidic protein) promoter is selected as a marker promoter for the starting cells, and a SYN (synapsin) promoter is selected as a marker promoter for the target cells. In another embodiment of the present disclosure, fibroblasts cells and neurons are selected as starting cells and target cells, respectively, and the fibroblasts are converted into the neurons. In this case, a Col1A2 (Collagen Type I Alpha 2 Chain) promoter is selected as a marker promoter for the starting cells.

After 72 hours, the medium was removed, the cells were concentrated for 72 hours by PEG-it Virus Precipitation Solution (LV810A-1, System Biosciences, USA) capable of concentrating a lentiviral vector, and then re-suspended in EMEM (Eagle's minimum essential medium), thereby constructing an advanced *in vivo* reprogramming system (A-IVR) which is a lentiviral vector for direct reprogramming according to the present disclosure.

### Example 2. Production and Identification of Neurons Converted from Neuroglial Cells

Neuroglial cells were dispensed into a 6-well plate at a density of 1×10⁵ cells/well and cultured with EMEM containing 10% FBS and 1% penicillin-streptomycin (PS). The lentiviral vector (A-IVR) constructed according to the method of Example 1 by selecting fibroblasts as starting cells and neurons as target cells was introduced into the cultured cells together with 10 µg/ml of Polybrene transfection reagent (TR-1003-G, Merck Millipore, USA). Polybrene increases the binding affinity between the virus and the target cell by neutralizing the electrical repulsion between the virus and the target cell membrane. After 3 days, the medium was replaced with EMEM containing 10% FBS and 1 µg puromycin, and cells into which the gene was not introduced were selected using puromycin for 1 week. After 1 week, the medium was replaced with DMEM/F12: neurobasal (2:1) containing 0.8% N₂, 0.4% B27 and 1% PS, and then conversion into neurons was induced for 5 weeks while the medium was replaced once every 2 days.

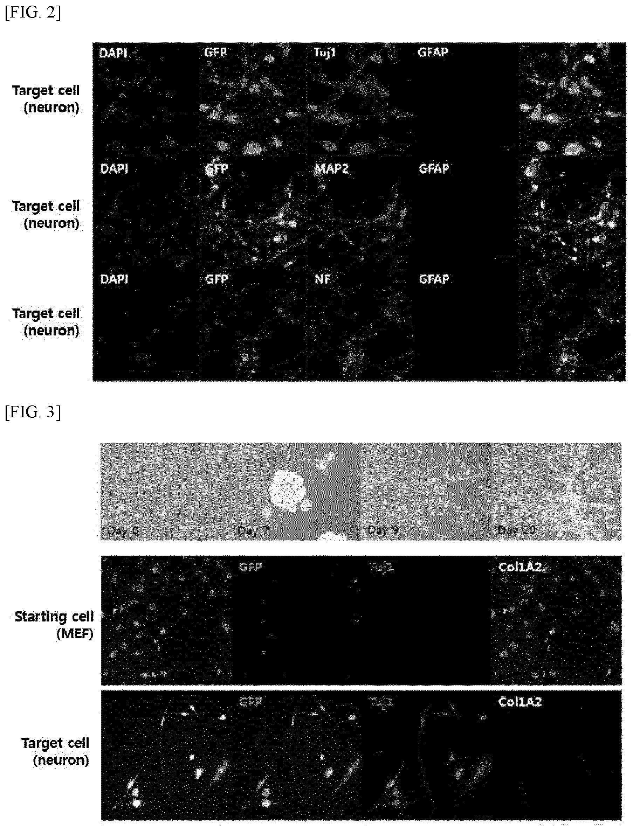

After 5 weeks, the cells were washed 3 times with PBS and fixed with 4% paraformaldehyde for 30 minutes. Next, the cells were washed three times with 0.3% Tween20 and then blocked using 10% normal donkey serum, followed by immunostaining with a neuroglial cell marker (GFAP) and neural markers (Tuj 1, MAP2, Neurofilament). The specific process of immunostaining was performed in the same manner as in the prior literature (Lee HY et al. Tissue Eng Part A. 2015 Jul; 21(13-14):2044-52). The results of the immunostaining are shown in FIG. 2.

As a result of the experiment, it was shown that, when the conversion of neuroglial cells, more specifically, astrocytes, into neurons, was induced using A-IVR *in vitro,* the expression of the neuroglial cell marker protein GFAP decreased and the neural marker proteins Tuj 1, Map2 and Neurofilament were expressed together with GFP. Thereby, it could be confirmed that neuroglial cells were converted into neurons by A-IVR, and GFP was also expressed along with neural differentiation.

### Example 3. Production and Identification of Neurons Converted from Fibroblasts

STO feeder cells (serving as a scaffold for cell growth) whose growth was inhibited by treatment with mitomycin C were seeded into a 12-well plate (coated with 0.1% gelatin) at a density of 2.5×10⁵ cells/well, and cultured with DMEM containing 10% FBS and 1% penicillin-streptomycin (PS) for 1 day. On the next day, mouse embryonic fibroblasts (MEF) or human fibroblasts were seeded onto the feeder cells at a density of 4×10⁵ cells/well and cultured with a DMEM or IMDM containing 10% FBS and 1% P/S for 1 day. Next, the lentiviral vector (A-IVR) constructed according to the method of Example 1 by selecting skin cells as starting cells and neurons as target cells was introduced into the cultured cells together with 10 µg/ml of Polybrene transfection reagent (TR-1003-G, Merck Millipore, USA). After 24 hours, the medium was replaced with DMDM/F12 containing 1% B27, 2 mM L-glutamate, 1% P/S, 20 ng/ml FGF, 20 ng/ml EGF, and 2 µg/ml heparin, followed by additional culture for 6 to 7 days. Thereafter, when a small colony was formed, the cells were detached using accutase (cell detachment solution) and further cultured in a 6-well plate for 3 days. The resulting sphere was transferred to a 0.1% gelatin-coated 12-well plate and cultured for 14 days to induce conversion into neurons.

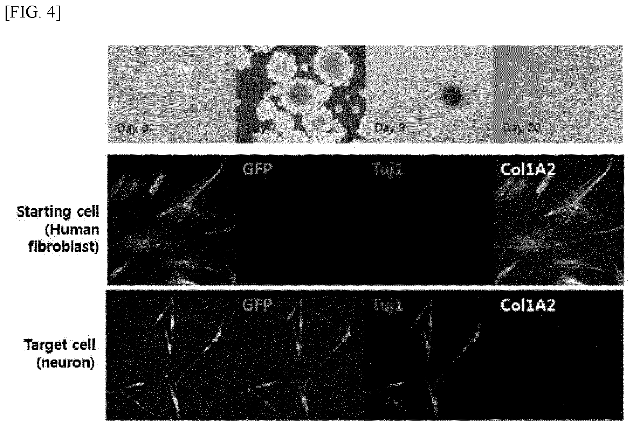

Immunostaining was performed in the same manner as in Example 2, except that a fibroblast marker (Col1A2) and a neural marker (Tuj 1) were used in the immunostaining. The results of the immunostaining are shown in FIGS. 3 and 4 together with optical micrographs of the cells.

As a result of the experiment, it was shown that, when the conversion of fibroblasts into neurons was induced using A-IVR *in vitro,* the expression of the fibroblast marker protein Col1A2 decreased, and the neural marker protein Tuj1 was expressed together with GFP. The above results were consistent regardless of the use of mouse embryonic fibroblasts or human fibroblasts as the starting cells. Thereby, it could be confirmed that the fibroblasts were converted into neurons by A-IVR, and GFP was also expressed along with neural differentiation.

### Example 4. Evaluation of Nerve Regeneration Effect in Spinal Cord Injury Animal Model Transplanted with A-IVR

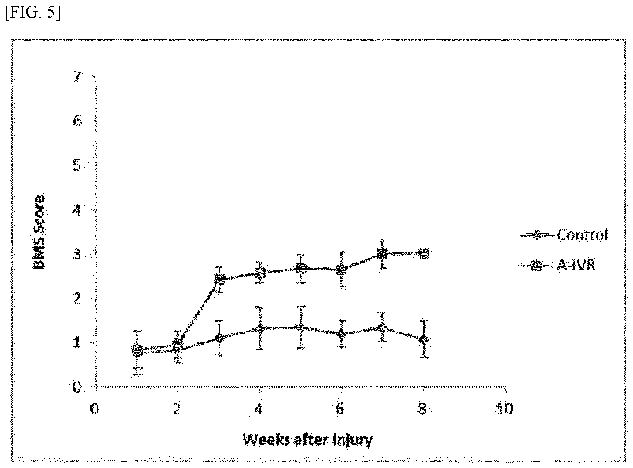

For construction of a spinal cord injury animal model, male C57BL/6 mice weighing about 20 g were used. Each mouse was anesthetized by intraperitoneal injection of a mixture of ketamine and Rompun, and thoracic vertebrae 10 (T10) was incised by laminectomy to expose the spinal cord, and then the spinal cord was injured by compressing the spinal cord using self-closed forceps (Germany) for 3 seconds. Thereafter, the muscles and skin were sutured. Two weeks after the construction of the spinal cord injury animal model, the skin and muscles of the injured site were opened again, and 4 µl of the lentiviral vector (A-IVR), constructed according to the method of Example 1 by selecting neuroglial cells (more specifically, astrocytes) as starting cells and neurons as target cells, was transplanted into the injured site by a 33G Hamilton syringe (n=10). Control mice were transplanted with EMEM instead of A-IVR (n=10). From one week after injury, Basso-Mouse-Scale (BMS) was measured weekly for motor function test. BMS is based on a 9-point scale and composed mainly of three steps. In the first step, the movement of the ankle is measured, and in second step, the weight-bearing gait and the recovery of the gait are measured, and in the last step, the stability of the gait and the recovery state of the tail are measured. The specific process of BMS was performed in the same manner as in the prior literature (Basso DM et al. J Neurotrauma. 2006 May; 23(5):635-59). The BMS measurement results are shown in FIG. 5. As a result of the experiment, it could be seen found that the motor function of the mice transplanted with A-IVR was significantly restored from 2 weeks after transplantation of the A-IVR compared to the control group.

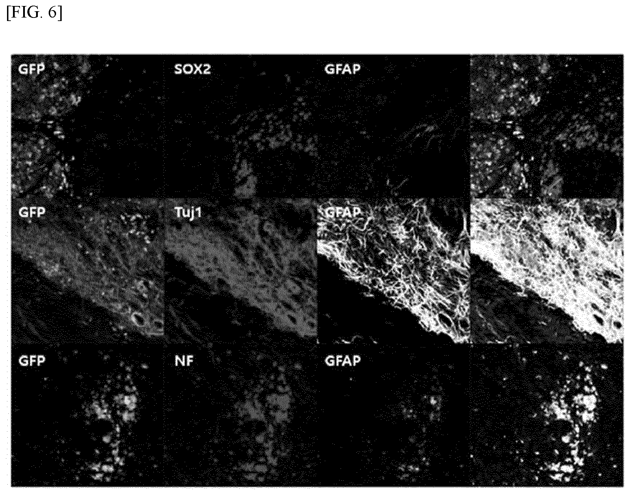

Eight weeks after spinal cord injury (6 weeks after A-IVR transplantation), the mice were sacrificed, and spinal cord tissue was isolated and fixed with 4% paraformaldehyde, followed by dehydration and freeze-embedding. The injured site (thoracic vertebrae 10) of the tissue subjected to embedding was immunostained with markers of neurons (MAP2), activated neuroglial cells (GFAP), neuroblasts (SOX2), early neurons (Tuj1), mature neurons (neurofilament), and the nuclei of all nucleated cells were counterstained with 4'6'-diamidino-2-phenylindole (DAPI, 1 µg/ml) (vector, CA, USA). The specific process of the immunostaining was performed in the same manner as in the prior literature (Lee HL et al. J Control Release. 2016 Mar 28; 226:21-34), and the results of the staining were visualized by laser scanning confocal microscopy (LSCM). The staining results are shown in FIG. 6. As a result of the experiment, it was confirmed that the neuroglial cell marker protein GFAP was expressed at and around the injured site. In addition, it was shown that the neuroblast marker protein Sox2 was not expressed together with GFP, but the early neural marker protein Tuj 1 and the mature neural marker protein Neurofilament were expressed together with GFP. GFAP was not co-expressed. Thereby, it could be seen that neuroglial cells were converted (reprogrammed) into neurons by A-IVR, and 6 weeks after vector introduction, the cells differentiated into neurons via neuroblasts.

From the results of Examples 1 to 4 above, it could be seen that the advanced *in vivo* reprogramming system of the present disclosure can convert (reprogram) starting cells into target cells, and confirm this conversion a living cell state. This means that necessary cells may be produced from other cells *in vivo.*

Although the present disclosure has been described in detail with reference to the specific features, it will be apparent to those skilled in the art that this description is only of a preferred embodiment thereof, and does not limit the scope of the present disclosure. Thus, the substantial scope of the present disclosure will be defined by the appended claims and equivalents thereto.

### Industrial Applicability

The present disclosure relates to an advanced *in vivo* reprogramming system and a cell conversion method using the same. The reprogramming system of the present disclosure does not require cell fixation to confirm cell conversion, enables real-time monitoring in a living cell state, and may be used both *in vitro* and *in vivo.* Thus, the reprogramming system of the present disclosure is expected to be widely used in the biological and medical fields.

## Claims

1. An expression vector for converting cell A into cell B, the expression vector sequentially containing a cell A marker promoter, a pluripotency maintaining gene protein, Cre recombinase, a cell B marker promoter, LoxP, and a gene encoding a fluorescent protein.

2. The expression vector of claim 1, which enables to confirm that cell A is converted into cell B in a living cell state.

3. The expression vector of claim 1, wherein the gene encoding the fluorescent protein is contained in a direction of reverse transcription.

4. The expression vector of claim 1, further comprising an amino acid isolation peptide.

5. The expression vector of claim 4, wherein the amino acid isolation peptide is any one or more selected from the group consisting of T2A, F2A, and E2A.

6. The expression vector of claim 1, wherein the pluripotency maintaining gene protein is any one or more selected from the group consisting of a SOX gene family (sex determining region gene family), a Myc gene family (proto-oncogene gene family), a Klf gene family (Kruppel-like factors gene family), and an Oct gene family (octamer-binding transcription factor gene family).

7. The expression vector of claim 1, wherein the cell A is a stromal cell, and the cell B is a neuron.

8. The expression vector of claim 7, wherein the stromal cell is any one selected from the group consisting of fibroblasts, chondroblasts, osteoblasts, neuroglial cells, adipocytes, macrophages, and plasma cells.

9. The expression vector of claim 7, wherein the cell A marker promoter is any one promoter selected from the group consisting of Col1A2 (Collagen Type I Alpha 2 Chain), FAP (fibroblast-activation protein), FSP1 (fibroblast-specific protein 1), vimentin, ACTA (alpha smooth muscle actin), Hsp47 (heat shock protein 47), aggrecan, CD44, CD45, CD73, calcitonin, OSCAR (osteoclast-associated receptor), RANK (receptor activator of nuclear factor κ B), GFAP (glial fibrillary acidic protein), TREM2 (triggering receptor expressed on myeloid cells 2), HexB (beta-hexosaminidase subunit beta), S100 (calcium-binding protein), CD69, and Gpr34 (probable G-protein coupled receptor 34).

10. The expression vector of claim 7, wherein the cell B marker promoter is any one promoter selected from the group consisting of SYN (synapsin), Tuj 1 (neuron-specific class III beta-tubulin), MAP2 (microtubule-associated protein 2), and Neurofilament.

11. A pharmaceutical composition for treating nerve injury containing the expression vector of claim 1 as an active ingredient.

12. A lentiviral vector containing the expression vector of claim 1, VSV-G (fusiogenic envelope G glycoprotein of the vesicular stomatitis virus), and a GAG/Pol gene.

13. A non-human transformant containing the expression vector of claim 1.

14. A method for converting cell A into cell B, the method comprising steps of:
(a) producing the expression vector of claim 1; and
(b) introducing the expression vector into cell A.

15. The method of claim 14, further comprising, after step (b), a step of culturing the cell with a cell B culture medium.

16. The method of claim 14, further comprising, after step (b), a step of confirming fluorescence expression in the cell.

17. A live cell imaging method for confirming the conversion of cell A into cell B in a living cell state, the method comprising steps of:
(a) producing the expression vector of claim 1; and
(b) introducing the expression vector into cell A.

18. The live cell imaging method of claim 17, wherein the imaging is performed using a fluorescence microscope.

19. A method for producing an animal model in which cell A has been converted into cell B, the method comprising step of:
(a) producing the expression vector of claim 1; and
(b) introducing the expression vector into a non-human subject.

20. A method for screening cell A for conversion into cell B, the method comprising steps of:
(a) preparing a first subject and a second subject as a non-human disease animal model;
(b) producing expression vector I of claim 1 by selecting cell (i) as cell A and cell (iii) as cell B;
(c) producing expression vector II of claim 1 by selecting cell (ii) as cell A and cell (iii) as cell B;
(d) introducing expression vector I into the first subject, and introducing expression vector II into the second subject; and
(e) comparing a disease therapeutic effect between the first subject and the second subject, and selecting cell (i) as a cell for conversion into cell (iii) when the therapeutic effect on the first subject is better.

21. A pharmaceutical composition for treating a disease caused by damage to cell B containing the expression vector of claim 1 as an active ingredient.

22. The pharmaceutical composition of claim 21, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.

23. A method of preventing or treating a disease caused by damage to cell B by administering the vector of claim 1 as an active ingredient.

24. The method of claim 23, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.

25. A use of the vector of claim 1 for preventing or treating a disease caused by damage to cell B.

26. The use of claim 25, wherein the cell B is a neuron, and the disease caused by damage to cell B is any one selected from the group consisting of epilepsy, amyotrophic lateral sclerosis (Lou Gehrig's disease), meningitis, encephalomeningitis, cerebral palsy, encephalitis, stroke, cerebral infarction, cerebral hemorrhage, ischemic brain attack, multiple sclerosis, headache, migraine, tension headache, chorea, Huntington's disease, Wilson's disease, concussion, brain contusion, subdural hematoma, traumatic subarachnoid hematoma, spinal cord injury, arteriovenous malformation, cerebral aneurysm, hydrocephalus, spina bifida, sleep apnea syndrome, syncope, nerve paralysis, severe asthenia, tremor, myelitis, Alzheimer's, Parkinson's disease, and motor dysfunction.
